# EUROPÄISCHE PATENTANMELDUNG

(11) **EP 0 934 725 A1**
(43) Veröffentlichungstag der Anmeldung: **11.08.1999**
(21) Anmeldenummer: 99102052.0
(22) Anmeldetag: 02.02.1999
(51) Int. Cl.: A61B 17/06

(54) **Chirurgische Nadeln mit Goldbeschichtung**

(30) Priorität: 04.02.1998 DE 19804246
(71) Anmelder: Fumedica Intertrade AG, 6331 Hünenberg (CH)
(72) Erfinder: Frenzel, Henry, 79798 Jestetten (DE)
(74) Vertreter: Patentanwälte Lippert, Stachow, Schmidt & Partner

(57) **Zusammenfassung**

Werden Nadeln für die Herstellung von Nähten in der Chirurgie zumindest im Bereich ihrer Spitzen vergoldet, dann wird ihre Penetrations- und Gleitfähigkeit verbessert.

## Beschreibung

Die Erfindung betrifft Nadeln zur Verwendung bei der Herstellung von Nähten in der Chirurgie, insbesondere in der allgemeinen sowie in der Herz-, Gefäß- und Mikrochirurgie.

Die beim Zusammenfügen von Wundrändern in der Chirurgie durch Nähen verwendeten Nadeln müssen eine Reihe verschiedener Anforderungen genügen. Die dabei angewendeten technischen Mittel wirken dabei oft nicht bei allen Anforderungen gleich günstig, sodaß der Chirurg auf Kompromisse angewiesen ist. Eine wichtige Forderung ist eine gute Penetrationsfähigkeit der Nadel, d.h. daß sie ohne großen Kraftaufwand das Gewebe durchdringen kann. Andererseits soll der durch die eindringende Nadel beschädigte Bereich des Gewebes möglichst klein sein. So dürfen bei der Gefäßchirurgie keine blutenden Stichkanäle in der Gefäßwand entstehen. Diese Forderungen versucht man durch geeignete Auswahl der Nadeln nach ihrer Geometrie, Größe, Formung des Nadelkörpers, der Nadelspitze und der Fadenaufnahme sowie der Oberflächenbeschaffenheit soweit möglich zu erfüllen.

Die Penetrationsfähigkeit von Nadeln kann durch Erhöhung der Gleitfähigkeit mittels Glättung und Siliconisierung der Nadeloberfläche verbessert werden. Nadeln mit glatten Oberflächen neigen jedoch zum Festsaugen am Gewebe, wodurch der erforderliche Kraftaufwand und die Gefahr weiträumiger Verletzungen erhöht werden. Deshalb ist es üblich, die Nadeloberfläche aufzurauhen. Dadurch wird jedoch die Penetrations- und Gleitfähigkeit wieder vermindert. Es besteht daher das Bedürfnis nach weiterer Verbesserung dieser Eigenschaften.

Es ist bekannt, chirurgische Instrumente mit Goldüberzügen zu versehen, um ihnen Korrosionsbeständigkeit zu verleihen (Ullmanns Encyklopädie der technischen Chemie, 3. Auflage 1956, 7. Band, Seite 823). US-A-2865376 beschreibt ein Verfahren zum stromlosen Vergolden von chirurgischen Nadeln aus Kohlenstoffstahl zum Schutz vor Korrosion. Mit der Verwendung korrosionsbeständiger Werkstoffe ist dies jedoch überflüssig geworden.

Aufgabe der Erfindung ist es, chirurgische Nadeln anzugeben, die eine verbesserte Penetrations- und Gleitfähigkeit gegenüber bekannten Nadeln aufweisen.

Diese Aufgabe wird durch Nadeln nach dem Anspruch 1 gelöst. Es wurde überraschenderweise gefunden, daß Nadeln, die zumindest im Bereich der Nadelspitze mit einem Goldüberzug versehen sind, besser in das zu nähende Gewebe eindringen und in diesem gleiten, als Nadeln ohne Vergoldung. Dieser Vorteil zeigt sich sowohl bei vollständig vergoldeten Nadeln als auch bei solchen, die nur im Bereich der geschliffenen Nadelspitze bis geringfügig über die Schliffstelle hinaus vergoldet sind. Die Goldschicht ist bevorzugt so dick, daß sie gut sichtbar ist. Das entspricht einer Dicke von mindestens 0,3 bis etwa 0,5 µm.

Korrosionsbeständige Federstähle sind insbesondere Stähle mit hoher Zugfestigkeit und Elastizität, die durch bestimmte Legierungsbestandteile (beispielsweise mindestens 12 Gew. -% Chrom) beständig gegen Feuchtigkeit, Luftsauerstoff und aggressive Stoffe gemacht wurden. Die Stähle der erfindungsgemäßen Nadeln enthalten bevorzugt auch noch mindestens 7 Gew.-% Nickel.

Besonders bevorzugt sind Nadeln, deren Oberfläche vor der Vergoldung aufgerauht wurde, beispielsweise mit einer Rauhtiefe von 1 bis 2 µm. Die Oberfläche ist dabei bevorzugt hammerschlagartig aufgeworfen und somit glatt-strukturiert.

Die Nadelspitze kann eine übliche Form aufweisen, beispielsweise rund, schneidend sowie ggf. asymmetrisch trocarförmig; bevorzugt sind trocarförmige Spitzen.

Vorteilhaft ist die Oberfläche der erfindungsgemäßen Nadeln mit einem Silicon-Gleitmittel behandelt.

Die Größe der Nadeln kann je nach der zu verwendenden Fadenstärke und dem Einsatzgebiet zwischen wenigen mm für die Mikrochirurgie bis zu einigen cm für allgemeinchirurgische Eingriffe liegen. Entsprechend kann die Stärke der Nadel zwischen etwa 50 µm und einigen mm betragen.

Die Nadeln können gerade oder in üblicher Weise gebogen sein, beispielsweise 3/8, 1/2 oder asymptotisch.

Das Auftragen des Goldüberzugs geschieht bevorzugt mit Verfahren, die keine Erhitzung des Nadelkörpers erfordern, beispielsweise durch galvanische Vergoldung. Zur Erhöhung der Abriebfestigkeit der Goldschicht können dieser Anteile anderer Metalle einverleibt werden.

Durch den Goldüberzug ist es auch möglich, Beschädigungen der Nadeloberfläche leicht zu erkennen. Dadurch wird die Qualitätsprüfung bei der Herstellung und vor der Verwendung erleichtert. Außerdem werden Teilchen im Größenbereich von Mikrometern und darunter, die bei der Herstellung z. B. durch Schleifen entstehen können, und an der Nadeloberfläche haften, fixiert und können nicht beim Nähen in das Gewebe des Patienten eindringen. Schließlich werden auch allergische Reaktionen aufgrund des Nickel-Gehaltes des Nadelmaterials vermieden.

## Patentansprüche

1. Chirurgische Nadeln aus korrosionsbeständigem Federstahl, insbesondere zur Verwendung in der Herz-, Gefäß- und Mikrochirurgie, **dadurch gekennzeichnet**, daß ihre Oberfläche mit einer Goldschicht überzogen ist.

2. Nadeln nach Anspruch 1, **dadurch gekennzeichnet**, daß die Goldschicht nur im Bereich der Nadelspitze vorhanden ist.

3. Nadeln nach einem der vorangehenden Ansprüche, **dadurch gekennzeichnet**, daß die Goldschicht eine Dicke von 0,3 bis 0,5 µm aufweist.

4. Nadeln nach einem der vorangehenden Ansprüche, **dadurch gekennzeichnet**, daß ihre Oberfläche eine Mikrorauhigkeit von 1 bis 2 µm aufweist.

5. Nadeln nach einem der vorangehenden Ansprüche, **dadurch gekennzeichnet**, daß ihre Spitze eine ggf. asymmetrische Trocarform hat.

6. Verfahren zur Herstellung von chirurgischen Nadeln aus korrosionsbeständigem Federstahl, **dadurch gekennzeichnet**, daß man die Nadelrohlinge vollständig oder im Bereich der Nadelspitze mit einer Goldschicht überzieht.

7. Verfahren nach Anspruch 7, **dadurch gekennzeichnet**, daß die Goldschicht durch galvanische Vergoldung aufgetragen wird.
